# EUROPEAN PATENT SPECIFICATION

(11) **EP 3 531 990 B1**
(45) Date of publication and mention of the grant of the patent: **11.02.2026**
(21) Application number: 17792152.5
(22) Date of filing: 26.10.2017
(51) Int. Cl.: A61F 7/00, A61F 7/10, A61F 7/12, A61M 11/00, A61M 5/42, A61M 19/00

(54) **COOLING DEVICE**
KÜHLVORRICHTUNG
DISPOSITIF DE REFROIDISSEMENT

(30) Priority: 28.10.2016 GB 201618255
(43) Date of publication of application: 04.09.2019
(73) Proprietor: Whiteley, Mark, Guildford, Surrey GU2 7RF (GB); The Whiteley Clinic, Guildford, Surrey GU2 7RF (GB)
(72) Inventor: WHITELEY, Mark, Guildford, Surrey GU2 7RF (GB); NEMCHAND, Jaya, Guildford, Surrey GU1 2PT (GB)
(74) Representative: Carpmaels & Ransford LLP
(86) International application number: PCT/GB2017/053228
(87) International publication number: WO 2018/078371

(56) References cited:
- WO-A1-2011/159317
- WO-A1-2016/030707
- JP-A- 2010 284 330
- US-A- 5 628 769
- US-A1- 2004 181 269
- US-A1- 2010 049 126
- US-A1- 2011 098 791
- US-A1- 2015 223 975
- US-A1- 2016 242 956

## Description

### Field

The present invention relates to cooling devices and components thereof, and particularly, although not exclusively, to body surface cooling devices for cooling a region of a subject's body, be that internal or external, while treatment is taking place or prior to piercing it with a needle, scalpel, or other sharp object. The invention is especially concerned with reducing the temperature of a subject's skin or other area of the body prior to giving an injection or taking a blood sample, for example.

### Background

A great many medical and cosmetic interventions require the passage of a needle or similar device into or through the skin. For example, it is necessary for a hypodermic needle (i.e. a hollow needle) to pierce the skin of a patient to a sufficient depth to allow a medicament to be administered by an injection. It will be appreciated that the term injection typically encompasses intradermal (ID), intravenous (IV), intramuscular (IM), and subcutaneous (SC) administration, and the location of the depth to which the needle is inserted will depend upon the type of injection. Injections are amongst the most common healthcare procedures, with at least 16 billion administered in developing and transitional countries each year. 95% of injections are administered in curative care, 3% are for immunization, and the rest for other purposes, such as blood transfusions.

It will be appreciated that a hypodermic needle will also need to pierce the skin for a sample, such as blood, to be taken. Alternatively, a cannula may be used to administer and remove fluids instead of a syringe. A cannula generally comprises a short catheter (a few centimetres long) which is inserted through the skin into a vein. Usually, a flexible plastic cannula is provided mounted over a metal trocar. The tip of the trocar and cannula may pierce the skin and be introduced into the vein; the cannula may then be advanced inside the vein over the trocar to the appropriate position and secured. The trocar is then withdrawn and discarded. Medicament can then be delivered, or blood samples may be drawn directly from the patient, once the cannula has been inserted.

Finally, some procedures may involve solid needles, such as for electrolysis. In all of the above cases, the passage of a needle or similar devices through the tissue surface, such as the skin or oral mucosa (during dental procedures) is medically invasive and inherently causes pain. Specifically, in the case of skin, the pain receptors (nociceptors) in the dermis are stimulated when a needle or device passes into the dermis. Accordingly, pain relief may be required. The layer under the dermis, the subcutaneous fat, tends to lead to lesser observed pain when needles are passed into this area. However, if a substance that is an irritant is injected into this fat layer then pain can often result.

It is noted that since needles are often used to apply a small puncture wound to the body, with varying degrees of pain inherent to this, fear of needles is a common phobia. Numerous techniques are commonly used in an attempt to reduce the discomfort felt by the subject. For instance, a clinician may rub the tissue area prior to inserting a needle, utilising the "gate theory", in an attempt to reduce the pain. Alternatively, distraction techniques and topical local anaesthetics, usually left for upwards of 30 minutes, may be used.

Laboratory and clinical research have shown that cooling the surface of the skin to less than 10°C reduces the pain felt when the skin is stimulated by the passage of a needle or when other painful stimuli are applied thereto. It has been found that skin that has hair tends to more susceptible to anaesthesia by cooling and the effect lasts longer when the cooling is removed. Accordingly, ice is sometimes applied to the skin to reduce pain. The ice either needs to be contained in a sealed container or will lead to wetness as the ice melts. Moreover, the ice itself may cause damage to the skin.

In addition to these problems, there are also a number of heat treatments used in curative and aesthetic medicine that can cause damage if the heat spreads to a non-target area. For example, new approaches to curing excess underarm sweating rely on applying microwave radiation to the body. The microwaves are intended to penetrate the skin and cause water in cells beneath the skin to become excited (i.e. heated) and irreparably damaged. While damaging the cells beneath the skin is intentional, it is important that the outer layer of skin is not damaged by the heat. It can be difficult to actively heat one area while preventing the heat spreading to a non-target area.

A number of cooling devices have also been developed. Commonly these devices are in one piece. Additionally, other devices, such as a canister comprising a vapour cooling spray are entirely disposable and need to be constantly replaced, leading to an increase in cost. US2016/0242956 relates to a thermoelectric anaesthetic cooling device to allow for temperature controlled cooling of the skin or mucous membrane to alleviate pain associated with medical treatment such as injections and skin ablation.

US2011/0098791 relates to a probe for a local anaesthetic system, the probe comprising a cooling pad to contact the surface to be cooled.

JP2010284330 relates to a device cooled by a Peltier element for contacting the skin to cool the skin.

US2010/0049126 relates to an injector including a Peltier cooled cool place to cool the skin prior to piercing the skin with the needle.

US2004/181269 relates to a Skin cooling device using thermoelectric element.

The invention arises from the inventor's work in attempting to address the problems associated with the prior art.

### Summary

According to the present invention, there is provided a cooling device according to claims 1 and 15. Methods of treatment disclosed here below are not claimed but are useful for the understanding of the invention.

According to a first aspect of the present disclosure; there is provided a cooling device for cooling a subject's tissue or organ surface, the device comprising:
- a handle;
- a head comprising:
   - an electrical cooling element for cooling the subject's tissue or organ surface; and
- a controller configured to receive an initial temperature of the subject's tissue or organ surface; determine a cooling temperature based on the initial temperature of the subject's tissue or organ surface and a target temperature; and control the cooling element to generate the cooling temperature.

Advantageously, the cooling device provides a means to cool a subject's tissue or organ surface such that a needle or similarly sharp instrument can be inserted into the subject's tissue or organ surface painlessly.

According to a second aspect of the present disclosure, there is provided a head for a cooling device for cooling a subject's tissue or organ surface, the head comprising:
- an electrical cooling element for cooling a subject's tissue or organ surface; and
- an interface for coupling the head to a handle of a cooling device, such that the head can communicate with the handle.

Advantageously, the head can be discarded and another used in its place for a different patient, or for a different part of the body, without discarding the whole device to which it is attached.

According to a third aspect of the present disclosure, there is provided a handle for a cooling device for cooling a subject's tissue or organ surface, the handle comprising:
- an interface for coupling the handle to a head of the cooling device, such that the handle can communicate with the head; and
- a controller configured to:
   - receive an initial temperature of the subject's tissue or organ surface;
   - determine a cooling temperature based on the initial temperature of the subject's tissue or organ surface and a target temperature; and
   - control the cooling element to generate the cooling temperature.

Preferably, the head of the second aspect and/or the handle of the third aspect are attached together to form the cooling device of the first aspect. Preferably, the cooling device comprises a fixing means for removably attaching the head to the handle. Preferably, the fixing means comprises a hook disposed on one of the head and the handle and a groove disposed on the other one of the head and the handle. Alternatively, the fixing means comprises pins and corresponding receiving holes, or clips. Preferably, the controller is disposed in the handle.

The head may be integrally formed with the handle, and the head comprises a removable non-porous layer between the cooling element and the subject's tissue or organ surface. Even more preferably, the non-porous layer comprises an adhesive.

Preferably, the head is rotatable with respect to the handle, such that the head can contact the subject's tissue or organ surface while the handle is held away from the subject's tissue or organ surface. Preferably, the head is rotatable from about -90 ° to about +90° to the longitudinal axis of the handle.

The controller is preferably configured to determine a cooling time based on the initial temperature of the subject's tissue or organ surface, the target temperature, and the cooling temperature.

The cooling device preferably comprises a temperature sensor for measuring the temperature of the subject's tissue or organ surface.

Preferably, the head further comprises a fixing means for removably attaching the head to the handle. Even more preferably, the fixing means comprises a locking mechanism for fixing the angle of the head with respect to the handle such that the applying pressure to the handle applies pressure to the head.

Preferably, the head comprises at least one fin for dissipating heat generated by the cooling element.

Preferably, the shape of the cooling element substantially corresponds to the shape of the head. It will be appreciated that the size and shape of the head used will depend on the target site on the subject's body, and the nature of the procedure being carried out thereon.

A "subject" may be a vertebrate, mammal, or domestic animal. Hence, the cooling device may be used to treat any mammal, for example livestock (e.g. a horse), pets, or may be used in other veterinary applications. Most preferably, however, the subject is a human being.

The head is preferably annular defining an aperture, and through which a medical instrument, such as a needle, may be passed. The aperture formed by the annular head may be any shape, size and/or diameter to achieve the desired technical effect. Accordingly it can be, but is not limited to, a circular shape, whose dimensions are sufficient to accommodate a needle to be passed therethrough. The aperture can be square, rectangular, triangular, or diamond-shaped, for example.

In another embodiment, the head may comprise one or more spaced-apart curvilinear arms which mutually define an open-ended aperture. The aperture may, for example, have a V-shaped profile or U-shaped profile. This provides the user with unimpeded visibility of the target area.

Preferably, the head comprises a window in the cooling element for allowing light to pass through the head to the subject's tissue or organ surface. The window may comprise a transparent material such as glass, Perspex, or plastic. Even more preferably, the window comprises an air gap. Advantageously, the aperture or window acts as a guide via which the user can direct the head to the target zone on the subject's tissue or organ surface.

Preferably, the cooling element is configured to cool a subject's tissue or organ surface, when activated, to a temperature of between -30°C and 15 °C. More preferably, the cooling element is configured to cool the subject's tissue or organ surface to a temperature of between -25 °C and 10 °C. Most preferably, the cooling element is configured to cool the subject's tissue or organ surface to a temperature of between -20 ° C and 5 °C. Still more preferably, the cooling element is configured to cool the subject's tissue or organ surface to a temperature of between -15 °C and 0 °C. Most preferably, the cooling element is configured to cool the subject's tissue or organ surface to a temperature of between -10 °C and -5 °C. Preferably, the cooling element is configured to cool the subject's tissue or organ surface to a temperature below 5 °C, 0 °C, or -5 °C.

Preferably, the cooling element comprises a thermoelectric device using the Peltier effect. More preferably, layers of the thermoelectric device comprise bismuth and copper.

Preferably, the upper surface of the head comprises at least one vent for dissipating heat generated by the cooling element.

Preferably, at least the lower surface of the head is fabricated from an expandable material adapted to expand and contract with thermal expansion and/or contraction of the cooling element. More preferably, the lower surface is fabricated from a highly thermally conductive material to maximise thermal energy transfer from the cooling element to the subject's tissue or organ surface to which it is applied. Even more preferably, the lower surface is fabricated from fine gauge stainless steel, other metals, alloys or a similar non-metal material such that the lower surface is sufficiently durable to withstand sudden temperature changes.

Preferably, the lower surface of the head is substantially flat. Alternatively, the lower surface is either concave or convex or a combination of the two. For example, when treating significantly concave areas of the subject's tissue or organ surface, a convex surface is desirable in order that as much of the surface as possible can engage the subject's tissue or organ surface to provide effective contact with the subject's tissue or organ surface. Conversely, when treating significantly convex areas of the subject's tissue or organ surface, a concave surface is desirable.

Preferably, the head defines a hollow cylinder, and preferably the cooling element is disposed on the outer surface of the hollow cylinder such that the inside of a cylindrically-shaped vessel can be cooled.

Even more preferably, the inner surface of the hollow cylinder comprises at least one vent for dissipating heat generated by the cooling element.

Preferably, the head is disposable. Preferably, the head comprises a removable sterile cover.

Thus, preferably the target temperature is between -30 °C and 15 °C, more preferably between -25 °C and 10 °C, more preferably between -20 ° C and 5 °C, still more preferably between -15 °C and 0 °C, and most preferably between -10 degrees Celsius and -5 °C. Preferably, the target temperature is below 5 °C, 0 °C, or -5 °C. Preferably, the controller is configured to vary the cooling temperature.

Preferably, the controller is configured to turn off the cooling element when the target temperature is reached. Alternatively, the controller is configured to calculate a cooling time, and turn off the cooling element after the cooling time. According to the invention, the handle comprises a user input for selecting a subject's body part to be cooled, and the controller is configured to determine the cooling temperature based on the selected body part.

Preferably, the controller is configured to optimise the cooling temperature based on the cooling time, temperature of the subject's tissue or organ surface, and target temperature.

Preferably, the handle comprises a power source. More preferably, the power source is a battery. Alternatively, the power source is a power adapter for coupling the cooling device to a mains supply.

Preferably, the handle comprises a display for displaying at least one of: the remaining time before the target temperature is reached, the current temperature of the subject's tissue or organ surface, and an indication of whether the target temperature is reached. Additionally or alternatively, the handle comprises an alarm for indicating whether the target temperature is reached.

Preferably, the handle comprises a thermally insulating material.

Preferably, the handle comprises a catheter.

According to a fourth aspect of the present disclosure, there is provided a method of cooling a subject's tissue or organ surface, the method comprising:
- receiving the initial temperature of the subject's tissue or organ surface;
- determining a cooling temperature based on the initial temperature of the subject's tissue or organ surface and a target temperature; and
- controlling a cooling element to generate the cooling temperature.

Preferably, the initial temperature of the subject's tissue or organ surface is received from a temperature sensor. Alternatively, the initial temperature of the subject's tissue or organ surface is received from a storage means.

The subject's tissue or organ surface which is cooled may be internal, for example a mucous membrane inside the mouth, vagina, nasal cavity or anus, or the outer surface of an internal organ. Preferably, the subject's tissue or organ surface is external, for example any part of the skin or the eye. Advantageously, the device, head and handle may be used to reduce the temperature of at least a region of a subject's tissue or organ surface, be that internal or external, prior to piercing the region with a needle, scalpel or other sharp instrument.

Preferably, the target temperature is between -30 °C and 15 °C. More preferably, the target temperature is between -25 °C and 10 °C. Most preferably, the target temperature is between -20 °C and 5 °C. Still more preferably, the target temperature is between -15 °C and 0 °C. Most preferably, the target temperature is between -10 degrees Celsius and -5 °C. Preferably, the target temperature is below 5 °C, 0 °C, or -5 °C.

Preferably, the method comprises varying the cooling temperature.

Preferably, the method further comprises receiving an indication of a subject's body part to be cooled, and the determining a cooling temperature comprises determining a cooling temperature based on the initial temperature of the subject's tissue or organ surface, the target temperature and the body part.

Preferably, the method comprises determining a cooling time and optimising the cooling temperature based on the cooling time, initial temperature of the subject's tissue or organ surface, and target temperature.

Preferably, the method comprises turning off the cooling element when the target temperature is reached. Alternatively, the method comprises turning off the cooling element after the cooling element has been activated for the duration of the cooling time.

All features described herein (including any accompanying claims, abstract and drawings), and/or all of the steps of any method or process so disclosed, may be combined with any of the above aspects in any combination, except combinations where at least some of such features and/or steps are mutually exclusive.

### Brief Description of the Figures

Embodiments of the present invention will now be described, by way of example only, with reference to the accompanying drawings, in which:
Figure 1a is a side view of a first embodiment of a cooling device comprising a handle and a head;
Figure 1b is a top view of the cooling device shown in Figure 1a;
Figure 2 is a bottom view of one embodiment of the head of the cooling device;
Figure 3 is a bottom view of a second embodiment of the head;
Figure 4 is a perspective view of a mechanical interface between the head and the handle of the cooling device;
Figure 5 is a side view of a second embodiment of the cooling device;
Figure 6 is a system diagram of a cooling device according to an embodiment;
Figure 7 is a perspective view of an embodiment of a cooling element within the head of the cooling device; and
Figure 8 is a flow diagram illustrating a method of cooling a tissue or organ surface according to an embodiment.

### Detailed Description

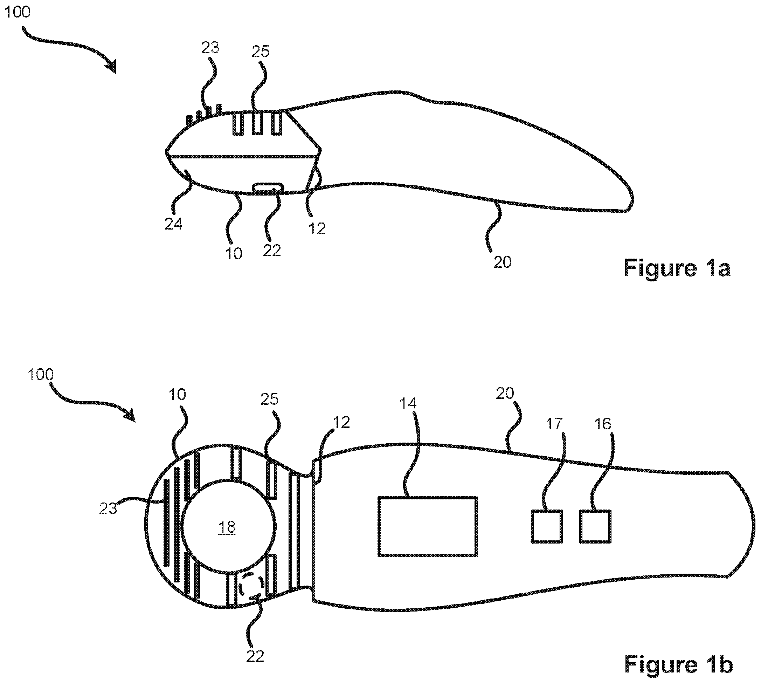

A cooling device 100, also known as a cryoanaesthesia device, shown in Figure 1, for use in reducing the temperature of a subject's tissue or organ surface prior to piercing it with a needle or other sharp instrument, such as a scalpel. The cooling device 100 can also be used to cool a part of a tissue or organ surface while another part of the tissue or organ surface is being actively heated. This prevents the heat from damaging unintended areas of the body tissue. The tissue or organ surface can be internal, such as the oral mucosal membrane (for use by dentists), a surface of an organ such an underside of the skin, or the inner membrane of the rectum or vagina, or external, such as the skin or the surface of the eye.

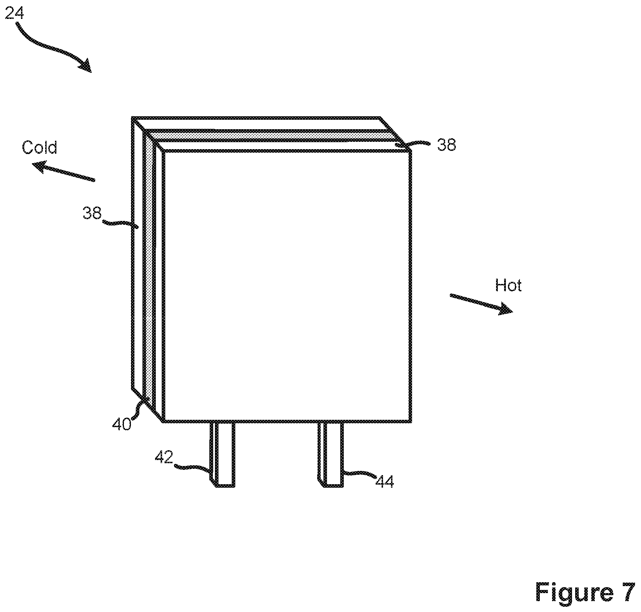

The device 100 comprises a head 10 connected to a handle 20. While the handle 20 shown in the Figures is an ergonomic design, in some embodiments the head 10 is connected to a catheter such that it can be inserted into the body. Generally, the head 10 comprises a cooling element 24 (shown in detail in Figure 7) and the handle 20 comprises control components for controlling the cooling element 24. The cooling element 24 according to an exemplary embodiment is a thermoelectric device which utilises the Peltier effect, described in more detail with reference to Figure 7. It would be appreciated that the cooling element 24 in other embodiments is any reusable cooling means. The handle 20 is ergonomically designed such that the device 100 is operable with one hand.

When working with humans or animals, it is essential to maintain a sterile environment. Particularly, if an instrument is not sterile, the bacteria that may have collected on the instrument can be transferred to a tissue or organ surface, and then infect a wound when the tissue or organ surface is punctured by a needle. Therefore, medical instruments need to be easy to sterilise, or designed for one-time use. Sterilising instruments typically involves heating the instrument using steam in an autoclave. Clearly, this is not appropriate for instruments having electrical components. Methods of sterilization also include using gamma radiation and Ethylene Oxide (EtO) treatment, but these are costly and labour-intensive.

Therefore, in one embodiment, the present invention provides a cooling device 100 having a disposable head 10. This prevents wastage of the handle 20 which has not come into contact with another human or animal.

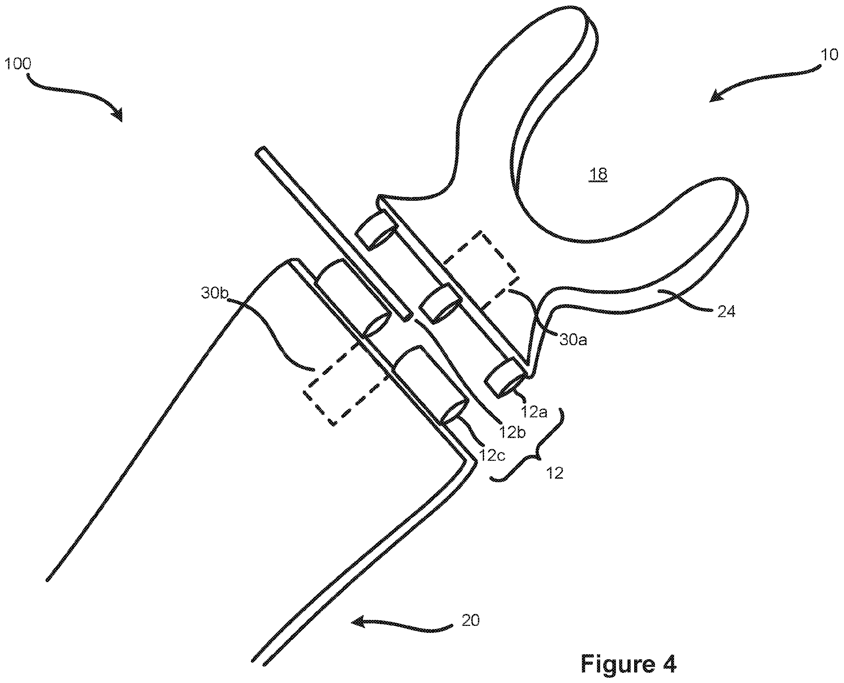

The head 10 is coupled to the handle 20 at a mechanical interface 12, which will be described with reference to Figure 4. The mechanical interface 12 allows the head 10 to rotate relative to the longitudinal axis of the handle 20. Preferably the mechanical interface 12 allows the head 19 to rotate between 0 ° and about +/-90° to its longitudinal axis. However, it would be readily understood that for some difficult to reach parts of the body, it is advantageous to have the head 10 back towards the handle 20 such that it faces the user. Here, the head 10 rotates between about -180 ° and about +180 ° to the longitudinal axis. It would be apparent in practice that the head 10 cannot rotate a full 180 ° to the longitudinal axis as the handle 20 will block its traverse. In some embodiments, the head 10 is coupled to the handle 20 such that it can twist (i.e. rotate in parallel to the longitudinal axis) and rotate (i.e. perpendicularly to the longitudinal axis) relative to the handle 20.

The mechanical interface 12 includes a locking mechanism (not shown) for fixing the angle of the head 10 relative to the handle 20. The locking mechanism may be a mechanical switch connected to a member to block the traverse of the head 10, or a resilient member such as a coil spring or cushion. Therefore, once the appropriate angle has been achieved, it can be fixed so that pressure can be applied to the head 10 through the handle 20.

The head 10, provided separately to the handle 20, is encased in a sterile air-tight package prior to use. The cooling element 24 is disposed at a lower portion of the head 10. The lower portion of the head 10 is the side of the head 10 closest to the subject's tissue or organ surface when the cooling device 100 is used. The tissue or organ surface may be, for example, the subject's skin, mucous membrane inside the mouth (oral mucosa) or genital region, anus, nasal cavity, or organs (internal or external) such as the eye or lungs.

As shown in Figure 1, an upper portion of the head 10 comprises at least one vent 25 for dissipating heat generated by the cooling element 24. The upper portion of the head 10 may also comprise fins 23 for increasing the surface area of the head 10, and therefore improve heat dissipation.

In some embodiments, the head 10 also includes a temperature sensor 22 for sensing the temperature of the tissue or organ surface. The temperature sensor 22 is any known means for detecting the temperature of an object, converting the temperature into temperature information and transmitting the temperature information electronically. For example, the temperature sensor 22 is a thermocouple. In other embodiments, the temperature sensor 22 is one of a Negative Temperature Coefficient thermistor, a Resistance Temperature Detector and a semi-conductor-based sensor. The temperature sensor 22 is formed on a lower side of the head 10, such that it is in close contact with the subject's tissue or organ surface when the cooling device 100 is in use.

In still other embodiments, the temperature sensor 22 is not necessary. Here, the body or organ tissue's initial temperature is assumed to be within a normal range known in the art. For example, the skin's surface temperature on a healthy human is about 34 degrees Celsius. This initial temperature, or temperature range from which the most conservative value is chosen for safety, may be received manually from a user input, or received from a storage means. An algorithm executed by a processor or controller in the cooling device 100 is used to determine the length of time and cooling temperature necessary to cool the tissue or organ surface to a target temperature from the initial temperature. The cooling process is determined to be complete when the determined time expires.

The cooling side of the cooling element 24 may form part of the outer surface of the head 10. Alternatively, the head 10 may comprise a housing, with the cooling element 24 disposed inside the housing.

When the cooling element 24 does not form the outer surface of the head 10, at least the lower surface of the head 10 is fabricated from an expandable material adapted to expand and contract with thermal expansion and/or contraction of the cooling element 24. The material is highly thermally conductive to maximise thermal energy transfer from the cooling element 24 to the tissue or organ surface to which it is applied. For example, the lower surface is fabricated from fine gauge stainless steel, other metals, alloys or a similar non-metal material such that the lower surface is sufficiently durable to withstand sudden temperature changes.

In some embodiments, the lower surface of the head 10, be it formed of the cooling element itself 24 or a separate housing layer, is substantially flat. Alternatively, the lower surface of the head 10 is either concave or convex or a combination of the two. For example, when treating significantly concave areas of the tissue or organ surface, a convex surface is desirable in order that as much of the surface as possible can engage the tissue surface to provide effective contact with the tissue surface. Conversely, when treating significantly convex areas of the tissue or organ surface, a concave surface is desirable.

In the embodiment shown in Figure 1b, the head 10 is annular. In other words, the head 10 forms the outside of an opening 18 through which a needle can be inserted. The opening 18 effectively provides a viewing area through which the target site on the subject's tissue or organ surface can be viewed, be that a vein or hair follicle etc., and also provides access for a needle. The cooling element 24 covers a significant proportion of the area of the head 10 which makes contact with tissue or organ surface. In use, the cooling device 10 may be removed from the tissue or organ surface prior to injection, or the needle may be inserted directly through the opening 18. The opening 18 and cooling element 24 are arranged such that nerves surrounding a target are cooled, but the target itself is not cooled. For example, a thread vein would be difficult to inject into if it had itself been cooled. However, by targeting the nerves around the vein, the vein remains coloured and therefore easy to identify.

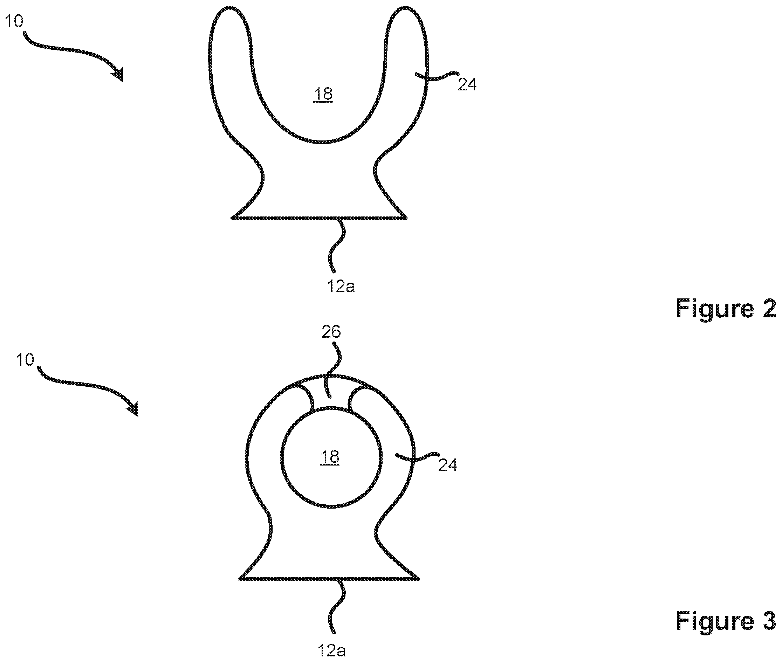

Head 10 shapes according to alternative embodiments are shown in Figures 2 and 3. In the embodiment of Figure 2, the head 10 does not form a circular aperture 18 at its centre. Instead, the aperture, or opening 18 is semi-circular created by two spaced apart curvilinear arms. This provides the user with unimpeded visibility of the target area.

An alternative approach to solve the same problem of improving visibility when using the device 100, without substantially reducing the cooling area, is shown in Figure 3. Here, a transparent window 26 is arranged to allow light to pass through the cooling element 24. The window 26 is arranged at the distal end of the head 10. In other embodiments, the window is disposed at the side or rear of the head 10. The window 26 is, for example, made of any suitable transparent material such as Perspex, plastic or glass. Replacing the window 26 with an air gap would effectively provide the design shown in Figure 2.

Alternatively to the shapes shown here, bespoke heads 10 tailored to specific body areas and injection sites may be used. For example, the opening 18 may be "V", "U" or "C" shaped to ensure accurate cooling.

Furthermore, the head 10 may be cylindrical so that it can easily be inserted into the body. Particularly, a cylindrical shape is useful when the cooling device 10 is used to cool the inner surface of a blood vessel or other tubular structure. The cylindrical head 10 may be a hollow cylinder, i.e. having an opening 18 at its central region along the longitudinal axis of the head 10. In these embodiments, the cooling element 24 may fully surround the outer surface of the head 10, while heat is dissipated into the head's 10 central region (i.e. the opening 18). The opening 18 may comprise vents and/or fins for dissipating heat energy away from the tissue or organ surface more effectively.

Returning to Figure 1b, the handle 20 comprises a display 14. The display 14 is, for example, an LCD display. In alternative embodiments, the display 14 is an LED display or an OLED display. The display 14 shows whether the tissue or organ surface (for example, skin) is at the appropriate temperature for injection. In other words, the display 14 provides an indication that the nerves around a target area have been sufficiently cooled such that the target area is numb. The display 14 may display the tissue or organ surface temperature directly, or a message indicating that the target temperature has been reached. The display 14 may flash to indicate the target temperature has been reached. In some embodiments, the display 14 displays the time left before the tissue or organ surface will reach the target temperature. The target temperature is typically between -10°C and -5°C. In other words, the target temperature is set to numb nerves, without causing damage to the tissue or organ surface, which may be internal or external to the body. Additionally, the target temperature can be set to keep the tissue or organ surface at a relatively constant temperature while surrounding tissue is heated.

The handle 20 of the cooling device 100 may also include a sound generator and/or an LED to indicate whether the desired target temperature has been reached.

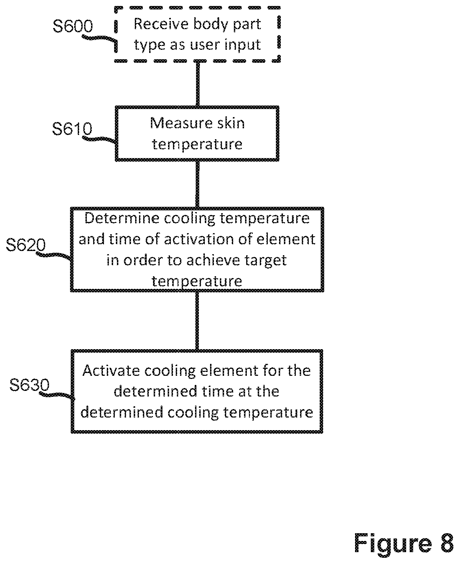

The handle 20 includes an on/off switch 16 for turning the device 100 on and starting the process described with reference to Figure 8. The cooling element 24 deactivates automatically when the target temperature is reached. If the device 100 is left switched on, the cooling element 24 reactivates when the tissue or organ surface temperature rises above a threshold temperature greater than the target temperature, in order to keep the temperature of the tissue or organ surface within a predetermined range.

The handle 20 also includes a user input 17 for selecting the body part having the tissue or organ surface which the user intends to cool. For example, the skin covering the thighs reacts differently to the skin around the eyes or the mucous membrane inside the mouth (oral mucosa), or the outer surface of an internal organ. The nerves may be denser, or buried deeper in the thighs, and so that the cooling element 24 needs to cool for longer. It may be the case that the area of skin is less sensitive, and so the cooling element 24 can output a lower cooling temperature in order to achieve the target temperature without causing discomfort.

The cooling device 100 may be used by a body piercer, for example, to reduce the pain cause by piercing a nostril. The cooling device 100 may also be used by a dentist, for example, to reduce the pain caused by injecting anaesthetic into a gum. The cooling device 100 may also be used, for example, by a cosmetic surgeon to reduce the pain caused by injecting collagen into lips. Furthermore, the cooling device 100 may be used with an electrolysis treatment. Electrolysis is the procedure for hair follicle removal using a fine, sterile and disposable needle to permanently destroy the follicle's ability to reproduce. This is achieved by destroying the hair making cells in the follicle wall of each hair, using chemical or heat energy. The cooling device 100 is applied to the skin before the fine needle is inserted in order to reduce the pain sensed when the needle is inserted.

The user input 17 shown in Figure 1b is a manual selector switch. However, in other embodiments, the display 14 is a touchscreen that provides a user input.

In some embodiments, the user input 17 provides a means to manually increase or decrease the temperature of the cooling element 24. The temperature of the cooling element 24 is also known as the cooling temperature.

An example of a mechanical interface 12 for coupling the head 10 to the handle 20 is shown in Figure 4. The interface 12 provides a means for attaching the head 10 to the handle 20, while allowing the head 10 to rotate relative to the handle 20. Primarily, this allows the head 10 to be swapped for a sterile head 10 between patients, or for a different type of head 10 for a different tissue or organ surface shape. Additionally, allowing the head 10 to rotate allows the handle 20 to be held comfortably away from the patient, while the head 10 remains in contact with the tissue or organ surface across its lower surface area.

A locking unit (not shown) fixes the angle of the head 10 with respect to the handle 20 once the desired angle has been achieved. This allows pressure applied to the handle 20 to be transmitted to the head 10 without causing further rotation. For example, the locking unit is a slider that slides over the interface 12 to prevent rotation. Additionally or alternatively to the locking unit, the interface 12 may be biased, for example using a coil spring such that it is resilient to movement.

In the embodiment shown in Figure 4, the interface 12 includes rings 12a disposed on the proximal end of the head 10. The interface 12 also includes rings 12c disposed on the distal end of the handle 20. The rings 12a of the head 10 and rings 12c of the handle 20 have a corresponding internal diameter. A pin 12b is used to pass through each of the rings 12a, 12c, thereby rotatably coupling the head 10 to the handle 20. The pin 12b can be pushed or pulled out of the rings 12a, 12c, so that the head 10 can be detached from the handle 20 without causing damage to the handle 20.

In other embodiments, the interface 12 comprises a universal joint, such that the head 10 can rotate and twist relative to the handle 20.

The rotatable aspect of the head 10 is not essential, and in other embodiments any well-known mechanical interface may be used to couple the head 10 to the handle 20 while allowing the head 10 to be later removed without causing damage to the handle 20. For example, one of the head 10 and handle 20 may include deformable hooks, and the other of the head 10 and handle 20 may include grooves or holes to receive the deformable hooks. The hooks are deformable such that they can be depressed to separate the head 10 from the handle 20, similarly to the clip on a bicycle helmet.

The cooling device 100 also includes an electrical interface 30. The electrical interface 30 includes two transceivers 30a, 30b, where one transceiver is disposed in the head 10 and the other is disposed in the handle 20. The electrical interface 30 provides a means for the head 10 to transmit temperature information from the temperature sensor 22 to the handle 20. Moreover, the electrical interface 30 provides a means to transfer power from a power source in the handle 20 to the cooling element 24 in the head 10.

The electrical interface 30 is a wired interface having a plug and socket type configuration. In other embodiments, the electrical interface 30 is a wireless transmitter, where magnetic coils are used to transfer electrical energy across a gap by inducing current.

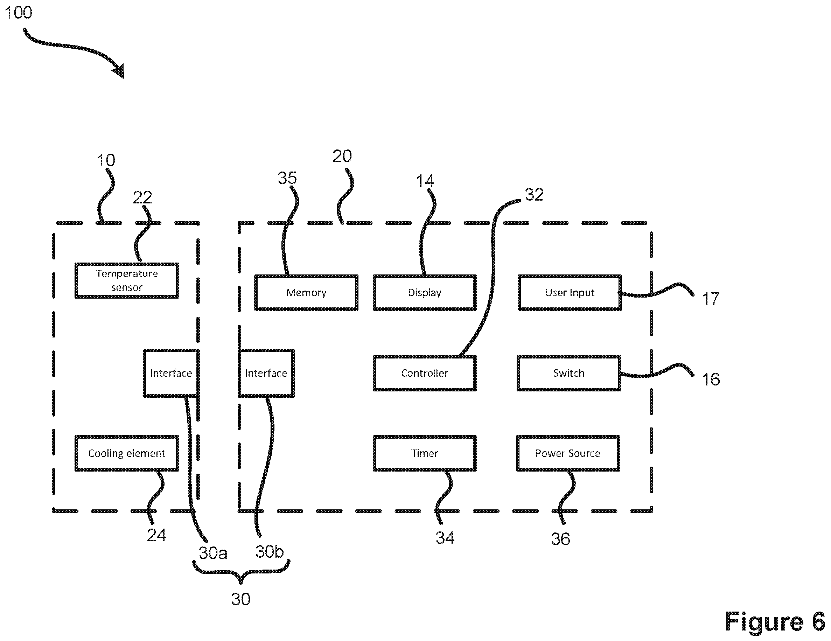

Further internal components of the handle 20 are will now be described with reference to Figure 6. A controller 32 such as a microprocessor is used to determine the length of time for which to active the cooling element 24. This is based on the initial temperature of the tissue or organ surface and the body part around which the tissue or organ surface is disposed. Alternatively, the time is pre-set. The time is set so that the tissue or organ surface is cooled quickly, but not so quickly as to cause shock. The length of time that the cooling element 24 has been in operation is determined from a timer 34. The timer 34 may be integrated with the controller 32.

The controller 32 also determines the amount of the current to supply to the cooling element 24 in order for the cooling element to be set at a particular temperature, known as the cooling temperature. The controller 32 can control the current by adjusting a variable resistor or a rheostat disposed between a power source 36 and the cooling element 24. The cooling temperature can be varied while cooling is taking place, until the temperature of the tissue or organ surface reaches a target temperature. The cooling temperature is optimised based on the cooling time and initial surface temperature such that the tissue or organ surface is effectively cooled without the patient feeling initial shock as the device 100 makes contact with the tissue or organ surface.

The memory 35 stores program instructions for allowing the controller 32 to function. For example, the memory 35 may be include a lookup table for associating tissue or organ surface temperatures with duration of cooling element 24 operation and cooling temperature.

The handle 20 also includes a power source 36 for driving the cooling element 24 in the head 10. The power source 36 according to some embodiments is a battery pack. In other embodiments, the power source 36 is a convertor for converting a mains power supply into a DC voltage for driving the cooling element 24.

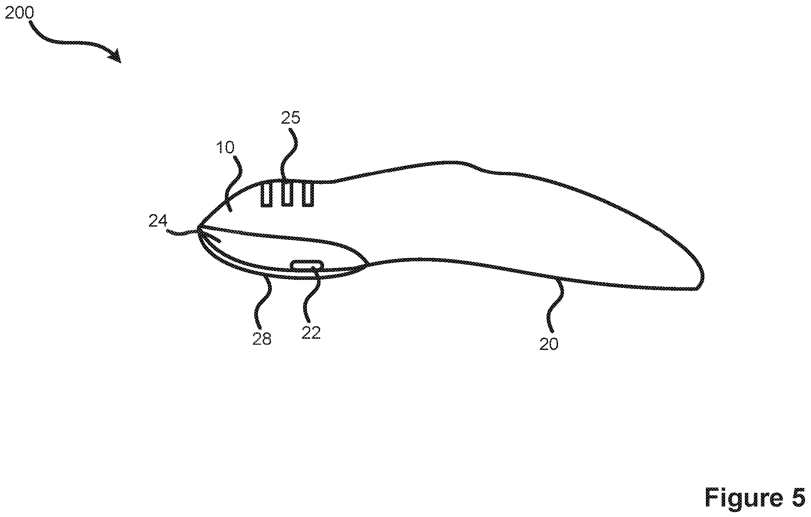

Figure 5 shows a cooling device 200 according to another embodiment. Here, the head 10 and handle 20 are integrally formed. To achieve the necessary degree of sterilisation, a sterile pad 28 is adhered to the distal end of the cooling device 200 at the region that will make contact with the tissue or organ surface. The sterile pad 28 may adhere to the cooling device 200 using an adhesive. Alternatively, the sterile pad 28 may be held in place using, for example, static electricity, Velcro^{™}, or a magnet.

The controller 32 is preprogramed with the thickness of the sterile pad 28, and is therefore able to compute the tissue or organ surface temperature using the temperature measured at the temperature sensor 22 which is separated from the tissue or organ surface by the sterile pad 28. Additionally, by being preprogramed with the thickness of the sterile pad 28, the controller 32 can calculate the cooling temperature required at the cooling element 24 to achieve the desired tissue or organ surface target temperature. In other embodiments, the user can select whether or not a sterile pad 28 is used, or specify the thickness of the sterile pad 28, using the user input 17.

The sterile pad 28 is made of a disposable material, and the cooling device 200 can be reused by attaching another sterile pad 28 to the device 200. The sterile pad 28 is non-porous, so that bacteria and liquids on the tissue or organ surface cannot soak through the sterile pad 28 and make contact with the lower surface of the cooling element 24.

The distal end of the cooling device 200 may take the shape of the heads 10 described with reference to Figures 1, 2 and 3. The sterile pad 28 may be cut by the user or preshaped to match the shape of the distal end of the cooling device 200.

Figure 7 shows a perspective view of a cooling element 24 according to an embodiment. The cooling element 24 is a thermoelectric device which uses the Peltier effect. The thermoelectric device may, for example, be a thermocycler, single stage, or multistage device. The thermoelectric device is a sandwich structure, with a first conductive material 40 being covered on both sides by a second conductive material 38. A positive electrode 42 and a negative electrode 44 protrude from one of the layers of second material 38 for receiving current from the power source 36 in the handle 20.

When two conductors are placed in electric contact, electrons flow out of the one in which the electrons are less bound, into the one where the electrons are more bound. The reason for this is a difference in the so-called Fermi level between the two conductors. The Fermi level represents the demarcation in energy within the conduction band of a metal, between the energy levels occupied by electrons and those that are unoccupied.

When two conductors with different Fermi levels make contact, electrons flow from the conductor with the higher level, until the change in electrostatic potential brings the two Fermi levels to the same value. Current passing across the junction results in either a forward or reverse bias, resulting in a temperature gradient. If the temperature of the hotter junction (heat sink) is kept low by removing the generated heat, the temperature of the cold plate can be cooled by tens of degrees.

The first conductive material 40 is an array of N- and P-type semiconductors. The thermoelectric semiconductor material most often used is an alloy of Bismuth Telluride (Bi2Te3) that has been suitably doped to provide individual blocks or elements having distinct "N" and "P" characteristics. Other thermoelectric materials include Lead Telluride (PbTe), Silicon Germanium (SiGe), and Bismuth-Antimony (Bi-Sb) alloys. The second conductive material 38 is for example a copper plate or an aluminium plate. Although not shown, ceramic plates may be disposed on the outside surface of at least one of the second conductive material 38 plates.

Figure 8 is a flowchart illustrating a method of cooling a tissue or organ surface using the cooling device 100. In a first optional step S600, the controller 32 receives an indication of the body part into which the user intends to inject. For example, the controller 32 may receive "thighs" through the user input 17.

The temperature sensor 22 then measures the initial tissue or organ surface temperature in step S610, and provides the temperature information corresponding to the temperature to the controller 32. Step 610 outlines just one example of receiving an initial surface temperature. In other embodiments, the initial tissue or organ surface temperature is assumed using a lookup table. Here, a storage means stores a list of tissues and organs and their corresponding normal temperature. The controller then looks up the necessary tissue or organ initial temperature when the tissue or organ is input by the user. For example, in a healthy human the skin surface temperature is about 34 degrees Celsius. In still other embodiments, the user can input the initial surface temperature manually using the user input 17.

In step S620, the controller 32 determines the cooling temperature based on the initial temperature of the tissue or organ surface, the target temperature and optionally the selected body part. The target temperature is the tissue or organ surface temperature required to numb the nerve endings, which may be disposed around 2.5 millimetres below the dermis, without causing damage to the tissue or organ surface. The cooling temperature is the temperature of the cooling element, which may be colder than or the same as the target temperature. The colder the cooling temperature, the quicker the tissue or organ surface reaches the target temperature. However, when working with humans and animals, it is not desirable to place an item that is too cold on a tissue or organ surface, or cool the tissue or organ surface too quickly, as this may shock the patient. Therefore, in some embodiments the controller 32 optimises the cooling time and cooling temperature according to the initial tissue or organ surface temperature, target temperature and optionally the selected body part. The target temperature is typically between -10 degrees Celsius and -5 degrees Celsius. However, in some specific cases the target temperature can be between -30 degrees Celsius and 15 degrees Celsius.

In step S630, the controller 32 controls the current provided to the cooling element 32 in order to set the cooling temperature. After the calculated time period, the controller 32 automatically turns off the cooling element 24.

In other embodiments, the tissue or organ surface temperature is measured at predetermined intervals, such as once per second, and the controller 32 turns off the cooling element 24 when the target temperature is reached.

Advantages of the cooling device 100, 200 of the invention reside in reducing the discomfort caused by piercing a tissue or organ surface (internal or external) with a needle or scalpel or the like. Moreover, the cooling device advantageously comprises a disposable element, so that the whole device does not need to be replaced between uses. Furthermore, by having interchangeable heads, the cooling device can be made bespoke for different areas of the body.

## Claims

1. A cooling device for cooling a subject's tissue or organ surface, the device comprising:
a handle (20);
a head (10) comprising an electrical cooling element (24) for cooling the subject's tissue or organ surface;
a user input (17) for selecting the subject's body part to be cooled; and
a controller (32) configured to:
determine a cooling temperature based on the selected body part; and
control the cooling element to generate the cooling temperature.

2. The cooling device according to claim 1, wherein the controller is disposed in the handle; and/ or the head is integrally formed with the handle, and wherein the head comprises a removable non-porous layer (28) between the cooling element and the subject's tissue or organ surface, optionally wherein the non-porous layer comprises an adhesive.

3. The cooling device according to any one of the preceding claims, wherein the head is rotatable with respect to the handle, such that the head can contact the subject's tissue or organ surface while the handle is held away from the subject's tissue or organ surface, optionally wherein the head is rotatable from about -90 ° to about +90 ° to the longitudinal axis of the handle.

4. The cooling device according to any of the preceding claims, comprising:
a temperature sensor (22) for measuring the temperature of the subject's tissue or organ surface; and/ or
at least one fin (23) for dissipating heat generated by the cooling element; and/ or
a window (26) in the cooling element for allowing light to pass through the head to the subject's tissue or organ surface, optionally wherein the window comprises a transparent material or an air gap.

5. The cooling device according to any one of the preceding claims, wherein the head is annular and defines an aperture through which a medical instrument can be passed, or the head comprises one or more spaced-apart curvilinear arms which mutually define an open-ended aperture.

6. The cooling device according to any one of the preceding claims, wherein the cooling element is configured to cool a subject's tissue or organ surface, when activated, to a target temperature of between -30 °C and 15 °C, or between -15 °C and 0 °C, or between -10 °C and -5 °C.

7. The cooling device according to any one of the preceding claims, wherein the cooling element comprises a thermoelectric device using the Peltier effect, optionally wherein layers of the thermoelectric device comprise bismuth and copper.

8. The cooling device according to any one of the preceding claims, wherein the upper surface of the head comprises at least one vent (25) for dissipating heat generated by the cooling element, and/ or wherein at least the lower surface of the head is fabricated from an expandable material adapted to expand and contract with thermal expansion and/ or contraction of the cooling element, and/ or the lower surface of the head is fabricated from a highly thermally conductive material to maximise thermal energy transfer from the cooling element to the subject's tissue or organ surface to which it is applied, and/or the lower surface of the head is one of concave, convex or a combination of the two.

9. The cooling device according to any one of claims 1 to 8, wherein the head defines a hollow cylinder, and wherein the cooling element is disposed on the outer surface of the hollow cylinder such that the inside of a cylindrically-shaped vessel can be cooled, optionally wherein the inner surface of the hollow cylinder comprises at least one vent for dissipating heat generated by the cooling element.

10. The cooling device according to any one of the preceding claims, wherein the head is disposable and/or comprises a removable sterile cover.

11. The head according to any one of the preceding claims, wherein the controller is further configured to; receive an initial temperature of the subject's tissue or organ surface; and determine a cooling temperature based on the initial temperature of the subject's tissue or organ surface and a target temperature

12. The cooling device according to claim 11, wherein the controller is configured to determine a cooling time based on the initial temperature of the subject's tissue or organ surface, the target temperature, and the cooling temperature, and/or the shape of the cooling element substantially corresponds to the shape of the head.

13. The cooling device according to any one of claims 11 or 12, wherein the controller is configured to:
vary the cooling temperature; and/ or
turn off the cooling element when the target temperature is reached; and/ or
calculate a cooling time, and turn off the cooling element after the cooling time; and/or
determine the cooling temperature based on the cooling time, temperature of the subject's tissue or organ surface, and target temperature.

14. The cooling device according to any one of claims 11 to 13, wherein the handle comprises:
a power source (36); and/or
a display (14) for displaying at least one of: the remaining time before the target temperature is reached, the current temperature of the subject's tissue or organ surface, and
an indication of whether the target temperature is reached; and/ or
an alarm for indicating whether the target temperature is reached; and/ or
a thermally insulating material; and/ or
a catheter.

15. A cooling device for cooling a subject's tissue or organ surface, the device comprising:
a handle (20);
a head (10) comprising an electrical cooling element (24) for cooling the subject's tissue or organ surface;
a user input (17) for selecting the subject's body part to be cooled; and
a controller (32) configured to:
receive an initial temperature of the subject's tissue or organ surface;
determine a cooling time based on the initial temperature of the subject's tissue or organ surface and the selected body part; and
control the cooling element to generate a cooling temperature.

## Patentansprüche

1. Kühlvorrichtung zum Kühlen einer Gewebe- oder Organoberfläche eines Subjekts, wobei die Vorrichtung umfasst:
einen Griff (20);
einen Kopf (10) umfassend ein elektrisches Kühlelement (24) zum Kühlen der Gewebe- oder Organoberfläche des Subjekts;
eine Benutzereingabe (17) zum Auswählen des zu kühlenden Körperteils des Subjekts; und
eine Steuerung(32), gestaltet zum:
Bestimmen einer Kühltemperatur auf der Grundlage des ausgewählten Körperteils; und
Steuern des Kühlelements zum Erzeugen der Kühltemperatur.

2. Kühlvorrichtung nach Anspruch 1, wobei die Steuerung in dem Griff angeordnet ist; und/oder der Kopf integral mit dem Griff gebildet ist, und wobei der Kopf eine entfernbare nichtporöse Schicht (28) zwischen dem Kühlelement und der Gewebe- oder Organoberfläche des Subjekts umfasst, wobei die nichtporöse Schicht gegebenenfalls einen Klebstoff umfasst.

3. Kühlvorrichtung nach einem der vorstehenden Ansprüche, wobei der Kopf bezogen auf den Griff drehbar ist, so dass der Kopf die Gewebe- oder Organoberfläche des Subjekts berühren kann, während der Griff von der Gewebe- oder Organoberfläche des Subjekts weg gehalten wird, wobei der Kopf gegebenenfalls von etwa -90° bis etwa +90° zu der Längsachse des Griffs drehbar ist.

4. Kühlvorrichtung nach einem der vorstehenden Ansprüche, umfassend:
einen Temperatursensor (22) zum Messen der Temperatur der Gewebe- oder Organoberfläche des Subjekts; und/oder
wenigstens eine Rippe (23) zum Abführen von durch das Kühlelement erzeugter Wärme; und/oder
ein Fenster (26) in dem Kühlelement, um zu ermöglichen, dass Licht durch den Kopf zu der Gewebe- oder Organoberfläche des Subjekts hindurchtritt, wobei das Fenster gegebenenfalls ein transparentes Material oder einen Luftspalt umfasst.

5. Kühlvorrichtung nach einem der vorstehenden Ansprüche, wobei der Kopf ringförmig ist und eine Öffnung definiert, durch die ein medizinisches Instrument geführt werden kann, oder der Kopf einen oder mehrere beabstandete gekrümmte Arme umfasst, die gemeinsam eine Öffnung mit offenem Ende definieren.

6. Kühlvorrichtung nach einem der vorstehenden Ansprüche, wobei das Kühlelement dafür gestaltet ist, die Gewebe- oder Organoberfläche eines Subjekts, wenn aktiviert, auf eine Zieltemperatur von zwischen -30 °C und 15 °C oder zwischen -15 °C und 0 °C oder zwischen - 10 °C und -5 °C zu kühlen.

7. Kühlvorrichtung nach einem der vorstehenden Ansprüche, wobei das Kühlelement eine thermoelektrische Vorrichtung, die den Peltier-Effekt einsetzt, umfasst, wobei gegebenenfalls Schichten der thermoelektrischen Vorrichtung Bismut und Kupfer umfassen.

8. Kühlvorrichtung nach einem der vorstehenden Ansprüche, wobei die obere Oberfläche des Kopfs wenigstens eine Lüftung (25) zum Abführen von durch das Kühlelement erzeugter Wärme umfasst und/oder wobei wenigstens die untere Oberfläche des Kopfs aus einem expandierbaren Material hergestellt ist, das dafür ausgelegt ist, sich mit Wärmeausdehnung und/oder - kontraktion des Kühlelements zu expandieren und zu kontrahieren, und/oder die untere Oberfläche des Kopfs aus einem gut wärmeleitfähigen Material hergestellt ist, um Wärmeenergieübertragung von dem Kühlelement auf die Gewebe- oder Organoberfläche des Subjekts, auf die er aufgebracht ist, zu maximieren, und/oder die untere Oberfläche des Kopfs eines von konkav, konvex oder eine Kombination der beiden ist.

9. Kühlvorrichtung nach einem der Ansprüche 1 bis 8, wobei der Kopf einen Hohlzylinder definiert und wobei das Kühlelement an der Außenfläche des Hohlzylinders angeordnet ist, so dass das Innere eines zylinderförmigen Behälters gekühlt werden kann, wobei gegebenenfalls die Innenfläche des Hohlzylinders wenigstens eine Lüftung zum Abführen von durch das Kühlelement erzeugter Wärme umfasst.

10. Kühlvorrichtung nach einem der vorstehenden Ansprüche, wobei der Kopf wegwerfbar ist und/oder eine entfernbare sterile Abdeckung umfasst.

11. Kopf nach einem der vorstehenden Ansprüche, wobei die Steuerung ferner dafür gestaltet ist, eine Anfangstemperatur der Gewebe- oder Organoberfläche des Subjekts zu empfangen und eine Kühltemperatur auf der Grundlage der Anfangstemperatur der Gewebe- oder Organoberfläche des Subjekts und einer Zieltemperatur zu bestimmen.

12. Kühlvorrichtung nach Anspruch 11, wobei die Steuerung dafür gestaltet ist, eine Kühlzeit auf der Grundlage der Anfangstemperatur der Gewebe- oder Organoberfläche des Subjekts, der Zieltemperatur und der Kühltemperatur zu bestimmen, und/oder die Form des Kühlelements im Wesentlichen der Form des Kopfs entspricht.

13. Kühlvorrichtung nach einem der Ansprüche 11 oder 12, wobei die Steuerung gestaltet ist zum:
Variieren der Kühltemperatur; und/oder
Abschalten des Kühlelements, wenn die Zieltemperatur erreicht ist; und/oder
Berechnen einer Kühldauer und Abschalten des Kühlelements nach der Kühldauer; und/oder
Bestimmen der Kühltemperatur auf der Grundlage der Kühldauer, der Temperatur der Gewebe- oder Organoberfläche des Subjekts und der Zieltemperatur.

14. Kühlvorrichtung nach einem der Ansprüche 11 bis 13, wobei der Griff umfasst:
eine Stromquelle (36); und/oder
eine Anzeige (14) zum Anzeigen von wenigstens einem von:
der verbleibenden Zeit vor Erreichen der Zieltemperatur, der aktuellen Temperatur der Gewebe- oder Organoberfläche des Subjekts, und
eine Anzeige, ob die Zieltemperatur erreicht ist; und/oder
einen Alarm zum Anzeigen, ob die Zieltemperatur erreicht ist; und/oder
ein wärmeisolierendes Material; und/oder
einen Katheter.

15. Kühlvorrichtung zum Kühlen einer Gewebe- oder Organoberfläche eines Subjekts, wobei die Vorrichtung umfasst:
einen Griff (20);
einen Kopf (10) umfassend ein elektrisches Kühlelement (24) zum Kühlen der Gewebe- oder Organoberfläche des Subjekts;
eine Benutzereingabe (17) zum Auswählen des zu kühlenden Körperteils des Subjekts; und
eine Steuerung(32), gestaltet zum:
Empfangen einer Anfangstemperatur der Gewebe- oder Organoberfläche des Subjekts;
Bestimmen einer Kühldauer auf der Grundlage der Anfangstemperatur der Gewebe- oder Organoberfläche des Subjekts und des ausgewählten Körperteils; und
Steuern des Kühlelements zum Erzeugen einer Kühltemperatur.

## Revendications

1. Dispositif de refroidissement pour refroidir un tissu ou une surface d'organe d'un sujet, le dispositif comprenant :
une poignée (20) ;
une tête (10) comprenant un élément de refroidissement électrique (24) pour refroidir le tissu ou la surface d'organe du sujet ;
une entrée utilisateur (17) pour sélectionner la partie corporelle du sujet à refroidir ; et
un dispositif de commande (32) configuré pour :
déterminer une température de refroidissement sur la base de la partie corporelle sélectionnée ; et
commander l'élément de refroidissement pour générer la température de refroidissement.

2. Dispositif de refroidissement selon la revendication 1, le dispositif de commande étant disposé dans la poignée ; et/ou la tête étant formée d'un seul tenant avec la poignée, et la tête comprenant une couche non poreuse amovible (28) entre l'élément de refroidissement et le tissu ou la surface d'organe du sujet, éventuellement la couche non poreuse comprenant un adhésif.

3. Dispositif de refroidissement selon l'une quelconque des revendications précédentes, la tête pouvant tourner par rapport à la poignée, de telle sorte que la tête peut entrer en contact avec le tissu ou la surface d'organe du sujet tandis que la poignée est maintenue à distance du tissu ou de la surface d'organe du sujet, éventuellement la tête pouvant tourner d'environ -90° à environ +90° par rapport à l'axe longitudinal de la poignée.

4. Dispositif de refroidissement selon l'une quelconque des revendications précédentes, comprenant :
un capteur de température (22) permettant de mesurer la température du tissu ou de la surface d'organe du sujet ; et/ou
au moins une ailette (23) pour dissiper la chaleur générée par l'élément de refroidissement ; et/ou
une fenêtre (26) dans l'élément de refroidissement pour permettre à la lumière de passer à travers la tête vers le tissu ou la surface d'organe du sujet, la fenêtre comprenant éventuellement un matériau transparent ou un espace d'air.

5. Dispositif de refroidissement selon l'une quelconque des revendications précédentes, la tête étant annulaire et définissant une ouverture à travers laquelle un instrument médical peut être passé, ou la tête comprenant un ou plusieurs bras curvilignes espacés les uns des autres qui définissent mutuellement une ouverture à extrémité ouverte.

6. Dispositif de refroidissement selon l'une quelconque des revendications précédentes, l'élément de refroidissement étant configuré pour refroidir un tissu ou une surface d'organe d'un sujet, lorsqu'il est activé, à une température cible comprise entre -30 °C et 15° C, ou entre -15 °C et 0° C, ou entre -10 °C et -5° C.

7. Dispositif de refroidissement selon l'une quelconque des revendications précédentes, l'élément de refroidissement comprenant un dispositif thermoélectrique utilisant l'effet Peltier, éventuellement les couches du dispositif thermoélectrique comprenant du bismuth et du cuivre.

8. Dispositif de refroidissement selon l'une quelconque des revendications précédentes, la surface supérieure de la tête comprenant au moins un évent (25) pour dissiper la chaleur générée par l'élément de refroidissement, et/ou au moins la surface inférieure de la tête étant fabriquée à partir d'un matériau expansible adapté pour se dilater et se contracter avec une dilatation et/ou une contraction thermique de l'élément de refroidissement, et/ou la surface inférieure de la tête étant fabriquée à partir d'un matériau hautement conducteur de la chaleur afin de maximiser le transfert d'énergie thermique de l'élément de refroidissement au tissu ou à la surface d'organe du sujet sur lequel elle est appliquée, et/ou la surface inférieure de la tête étant une surface concave, convexe ou une combinaison des deux.

9. Dispositif de refroidissement selon l'une quelconque des revendications 1 à 8, la tête définissant un cylindre creux, et l'élément de refroidissement étant disposé sur la surface extérieure du cylindre creux de telle sorte que l'intérieur d'un récipient de forme cylindrique peut être refroidi, éventuellement la surface intérieure du cylindre creux comprenant au moins un évent pour dissiper la chaleur générée par l'élément de refroidissement.

10. Dispositif de refroidissement selon l'une quelconque des revendications précédentes, la tête étant jetable et/ou comprenant un couvercle stérile amovible.

11. Tête selon l'une quelconque des revendications précédentes, le dispositif de commande étant en outre configuré pour : recevoir une température initiale du tissu ou de la surface d'organe du sujet ; et déterminer une température de refroidissement sur la base de la température initiale du tissu ou de la surface d'organe du sujet et d'une température cible.

12. Dispositif de refroidissement selon la revendication 11, le dispositif de commande étant configuré pour déterminer un temps de refroidissement basé sur la température initiale du tissu ou de la surface d'organe du sujet, la température cible et la température de refroidissement, et/ou la forme de l'élément de refroidissement correspondant sensiblement à la forme de la tête.

13. Dispositif de refroidissement selon l'une quelconque des revendications 11 ou 12, le dispositif de commande étant configuré pour :
faire varier la température de refroidissement ; et/ou éteindre l'élément de refroidissement lorsque la température cible est atteinte ; et/ou
calculer un temps de refroidissement et éteindre l'élément de refroidissement après le temps de refroidissement ; et/ou
déterminer la température de refroidissement sur la base du temps de refroidissement, de la température du tissu ou de la surface d'organe du sujet et de la température cible.

14. Dispositif de refroidissement selon l'une quelconque des revendications 11 à 13, la poignée comprenant :
une source d'énergie (36) ; et/ou
un dispositif d'affichage (14) permettant d'afficher au moins l'un des éléments suivants : le temps restant avant que la température cible soit atteinte, la température actuelle du tissu ou de la surface d'organe du sujet, et une indication indiquant si la température cible est atteinte ; et/ou
une alarme pour indiquer si la température cible est atteinte ; et/ou
un matériau isolant thermiquement ; et/ou un cathéter.

15. Dispositif de refroidissement pour refroidir un tissu ou une surface d'organe d'un sujet, le dispositif comprenant :
une poignée (20) ;
une tête (10) comprenant un élément de refroidissement électrique (24) pour refroidir le tissu ou la surface d'organe du sujet ;
une entrée utilisateur (17) pour sélectionner la partie corporelle du sujet à refroidir ; et
un dispositif de commande (32) configuré pour :
recevoir une température initiale du tissu ou de la surface d'organe du sujet ;
déterminer un temps de refroidissement sur la base de la température initiale du tissu ou de la surface d'organe du sujet et de la partie corporelle sélectionnée ; et
commander l'élément de refroidissement pour générer une température de refroidissement.
